# EUROPEAN PATENT SPECIFICATION

(11) **EP 0 877 250 B1**
(45) Date of publication and mention of the grant of the patent: **03.12.2003**
(21) Application number: 98106761.4
(22) Date of filing: 14.04.1998
(51) Int. Cl.: G01N 33/487, H01R 13/24

(54) **Dispensing instrument for fluid monitoring sensors**
Ausgabevorrichtung für Sensoren zur Überwachung von Flüssigkeiten
Dispositif de distribution pour des détecteurs de contrôle de fluides

(30) Priority: 28.04.1997 US 845685
(43) Date of publication of application: 11.11.1998
(73) Proprietor: Bayer Corporation, Pittsburgh, PA 15205-9741 (US)
(72) Inventor: Micinski, Russell J., South Bend, Indiana 46635 (US); Whitson, Robert C, Goshen, Indiana 46526 (US)
(74) Representative: Petrovicki, Wolfgang, Dr.

(56) References cited:
- EP-A- 0 764 469
- US-A- 4 203 203
- US-A- 5 026 290
- US-A- 5 096 669
- US-A- 5 199 882
- US-A- 5 575 403
- "ELASTOMERIC CONNECTORS MEET SMT REQUIREMENTS" EDN ELECTRICAL DESIGN NEWS,US,CAHNERS PUBLISHING CO. NEWTON, MASSACHUSETTS, vol. 34, no. 25, 7 December 1989 (1989-12-07), pages 74-79, XP000080809 ISSN: 0012-7515

## Description

### BACKGROUND OF THE INVENTION

### 1. Field of the Invention

The present invention generally relates to a fluid monitoring system, and, more particularly, to a new and improved instrument for handling multiple sensors that are used in analyzing blood glucose or other analytes contained therein and in particular, to electrically coupling certain of the electrical components within the instrument.

### 2. Background of the Invention

People suffering from various forms of diabetes routinely need to test their blood to determine the level of blood glucose. The results of such tests can be used to determine what, if any, insulin or other medication needs to be administered. In one type of blood glucose testing system, sensors are used to test a sample of blood.

Such a sensor may have a generally flat, rectangular shape with a front or testing end and a rear or contact end. The sensor contains biosensing or reagent material that will react with blood glucose. The testing end of the sensor is adapted to be placed into the fluid being tested, for example, blood that has accumulated on a person's finger after the finger has been pricked. A sufficient amount of fluid to be tested is drawn by capillary action into a capillary channel that extends between the mated pieces of the sensor from the testing end to the reagent material. The fluid then chemically reacts with the reagent material in the sensor with the result that an electrical signal indicative of the blood glucose level in the blood being tested is supplied to contact areas located near the rear or contact end of the sensor.

In order to couple the electrical signals produced at the sensor contacts to monitoring equipment, the sensors need to be inserted into sensor holders prior to the sensor end being placed into the fluid being tested. The holders have corresponding mating contact areas that become coupled to the contacts on the sensor when the sensor is inserted into the holder. Consequently, the holders act as an interface between the sensor and monitoring equipment that accumulates and/or analyzes the test results.

Prior to being used, the sensors need to be maintained at an appropriate humidity level so as to insure the integrity of the reagent materials in the sensor. Sensors have been packaged individually in tear-away packages so that they can be maintained at the proper humidity level. For instance, blister type packaging methods could be used. In this connection, the packages can include desiccant material to maintain the proper humidity or desiccate level in the package. In order for a person to use an individual sensor for testing blood glucose, the package must be opened by tearing the seal. Alternatively, some packages require the user to exert force against one side of the package resulting in the sensor bursting or rupturing the foil on the other side. As can be appreciated, the opening of these packages can be difficult. Moreover, once the package is opened, the user needs to be sure that the sensor is not damaged or contaminated as it is being placed into the sensor holder and used to test the blood sample.

Sensor dispersing instruments have been developed for dispensing individual ones of the sensors to a sensing or testing position from within a sensor pack loaded into the sensor dispensing instrument. One such type of sensor pack disclosed in US 5,5575,403 includes a generally circular shaped base portion in which is formed sensor retaining cavities or depressions sealed by a foil that is heat sealed about the cavities to isolate them from each other. Each of the sensor retaining cavities is adapted to receive one of the sensors and is in fluid communication with a corresponding desiccant cavity in which is disposed desiccant material. The desiccant material is placed in the cavity to insure that the corresponding sensor cavity is maintained at an appropriate humidity or desiccate level so that the reagent material in the sensor will not be adversely affected prior to the sensors being used.

The circular type sensor pack of US 5,575,403 can be loaded in a sensor dispensing instrument that has a feeding mechanism. When the feeding mechanism is actuated or moved forward toward a testing end of the instrument, one of the sensors in the sensor pack is ejected from the sensor pack and paced into a testing or sensing position with the testing end of the sensor projecting from the forward or testing end of the instrument. With the sensor in its testing position, the testing end of the sensor can be placed into the fluid (for example, blood) being tested and contacts in the instrument become mated with corresponding contacts on the sensor.

The sensor dispensing instrument needs to be relatively compact in size so that it can easily be carried out and handled by a person using the dispensing instrument. Even though it is relatively small in size, the sensor dispensing instrument not only has to include the mechanical elements needed to advance (rotate) the sensor pack disposed therein, to eject individual ones of the sensors from the individualized sensor cavities in the sensor pack and to move the ejected sensor into a testing position, but in addition it has to include a microprocessor or other data processing circuitry to obtain the data from the sensor during a test procedure, process inputs from manually actuated buttons to provide information with respect to the test being conducted and to provide the accumulated information to a display screen of the instrument and/or to other analyzing or computer equipment via a data port connector.

The sensor dispensing instrument according to US-A-5 575 403 comprises an outer housing, a display means in said housing for displaying information, a circuit means disposed in said housing adjacent said display means and having data processing means thereon to receive electrical data generated by the sensors, data means accessible from externally of said housing, and a connector means being disposed between the display means and the circuit means to couple said display means to said circuit means.

The data processing circuitry in US 5,575,403 is included on a printed circuit board disposed in the fluid sensor dispensing instrument. The circuits on the printed circuit board need to be coupled to the display screen as well as to the manually actuated input buttons and the data port connector. The limited amount of space available for the components within the compact sized fluid sensor dispensing instrument it difficult for the connections to be made to the circuitry on the printed circuit board from the manually actuated buttons, the data port connector and the display screen, particularly in view of the fact that the electrical contacts for the manually actuated input buttons and the data port connector extend in a direction that is generally parallel to the planes of the printed circuit board and the display screen.

### SUMMARY OF THE INVENTION

Accordingly, an object of the present invention is to provide a new and improved fluid sensor dispensing instrument handling device that is adapted to selectively place into a testing position individual sensors from a sensor pack disposed in the device and for obtaining data from tests conducted with those sensors and for providing the data and other information as to the test to a display screen and/or a data port connector. In particular, an object of the present invention is to provide a new and improved fluid sensor dispensing instrument handling device in which circuity on a printed circuit board disposed within the instrument is coupled by an elastomeric connector to a display screen that lies in a plane generally parallel to the plane of the printed circuit board and to manually actuated buttons and a data port connector which have contacts extending in a direction that is generally parallel to the planes of the printed circuit board and the display screen. This object is achieved with an instrument according to claim 1, additional features being set out in the dependent claims.

In accordance with these and many other objects of the present invention, the present invention is embodied in a sensor dispensing instrument such as disclosed in US 5,570,403 that is adapted to receive a generally circular shaped sensor pack containing a plurality of blood glucose sensors, each of which sensors is disposed in a sensor retaining cavity which in turn is in fluid communication with a corresponding desiccant cavity in which is disposed desiccant material such as dislosed in US 5,575,403. The sensor instrument includes an outer housing having an upper case and a lower case with the upper and lower cases being pivotable with respect to each other in a clam shell fashion so that the sensor pack can be positioned for use in the housing. With the sensor pack so disposed in the housing, a slide latch on a slide actuator disposed on the upper case of the housing controls the placing of the instrument into a display/data processing mode or in a testing mode.

The instrument is placed into its display mode by moving the slide latch to its display mode, lateral position on the slid actuator. When the slide actuator is pushed from its standby position near the rear of the instrument housing towards the forward end of the instrument housing, a person using the instrument can view data displayed on a display unit in the upper case and/or input data into the instrument via manually actuated buttons at the rear of the upper case. On the other hand, the instrument is in its testing mode when the slide latch is in its testing position and the slide actuator is pushed from its standby position towards its testing position near the forward end of the instrument housing. As the slide actuator is pushed toward its actuated or testing position, a sensor is ejected from one of the sensor cavities so that it is positioned in its testing position with the testing end of the sensor projecting out from the testing end of the housing and with contacts on the sensor coupled to a microprocessor and/or other data processing circuitry on a printed circuit board disposed within the upper clam shell of the outer housing. As a result, data obtained from the sensor when it is inserted into blood to be tested can be processed. The processed data then can be displayed on a screen in the upper case of the instrument or stored for later supplying via a data port connector to other analyzing equipment.

Once the blood analyzing test is completed, the slide actuator is moved in the opposite direction towards the rear of the instrument housing into its standby position. As the slide actuator is moved away from its testing position, the sensor is removed from the dispensing instrument and the sensor pack is advanced (rotated) so that another sensor can be ejected from the sensor pack and used in the next blood glucose test that is to be performed.

The dispensing instrument additionally includes user actuated buttons disposed adjacent the data port connector at the rear of the upper clam shell portion of the dispensing instrument housing. These buttons are used to permit the user of the instrument to input data to the electrical components on the printed circuit board or obtain information about the tests that are being conducted. Once data concerning the tests performed has been accumulated in the electrical circuitry on the printed circuit board, the data can be transmitted to other monitoring or computer equipment via the data port connector also mounted at the rear of the upper clam shell portion of the instrument housing.

The manually actuated buttons, the data port connector and the display screen all have to be electrically coupled to the circuitry on the printed circuit board. The connection of these components is somewhat complicated due to the limited amount of space within the fluid dispensing instrument and due to the fact that the display screen and the printed circuit board are in generally parallel planes and the contacts for the user buttons and the data port connector extend in a direction that is generally parallel to those planes. In order to accomplish these connections, an elastomeric connector having conductive traces or filaments on three sides thereof is sandwiched between the display screen and the printed circuit board with circuits on the display screen and printed circuit boards in contact with two opposite sides of the elastomeric connector and the user actuated buttons and data port connector making contact on a third side of the elastomeric connector. The elastomeric connector consists of a generally silicone sponge rubber core with U-shaped metal filaments wrapped around the core. The fine pitch between the metal filaments provides more conducting paths to be available for coupling the electrical components in the limited space within the fluid sensor dispensing instrument.

### BRIEF DESCRIPTION OF THE DRAWING

These and many other objects and advantages of the present invention will become readily apparent from consideration of the following detailed description of the embodiment of the invention shown in the accompanying drawing wherein:
FIG. 1 is a top plan view of a blood glucose sensor dispensing instrument embodying the present invention.
FIG. 2 is a side plan view of the blood glucose sensor dispensing instrument of FIG. 1;
FIG. 3 is a bottom plan view of the blood glucose sensor dispensing instrument of FIG. 1;
FIG. 4 is a rear plan view of the blood glucose sensor dispensing instrument of FIG. 1;
FIG. 5 is an exploded perspective view of a portion of the upper housing of the blood glucose sensor dispensing instrument of FIG. 1 viewed from the under side of the upper housing in order to illustrate manually actuated buttons, a data port connector, a printed circuit board, a display screen and a connector for coupling those components together;
FIG. 6 is an exploded perspective view of a portion of the upper housing of the blood glucose sensor dispensing instrument of FIG. 1 viewed from the under side of the upper housing in order to illustrate manually actuated buttons, a data port connector, a display screen that is mounted on a housing guide, and connectors for coupling those components to electronic circuitry on a printed circuit board (not shown in FIG. 6);
FIG. 7 is an exploded perspective view of components from the blood glucose sensor dispensing instrument of FIG. 1 showing manually actuated buttons, a data port connector, a printed circuit board, a display screen, and a connector for coupling those components together;
FIG. 8 is a side cross-sectional view of components from the blood glucose sensor dispensing instrument of FIG. 1 showing how a manually actuated button, the printed circuit board, the display screen, are coupled together by connectors;
FIG. 9 is an exploded perspective view of a sensor pack used in the blood glucose sensor dispensing instrument of FIG. 1 with the foil portion of the sensor pack separated from the base portion of the sensor pack and with a sensor disposed in each of the sensor cavities; and
FIG. 10 is a perspective view of a portion of the connector illustrated in FIGS. 5-7 used to couple manually actuated buttons, a data port connector, a printed circuit board, and a display screen together within the blood glucose sensor dispensing instrument of FIG. 1.

### DETAILED DESCRIPTION OF THE PREFERRED EMBODIMENT

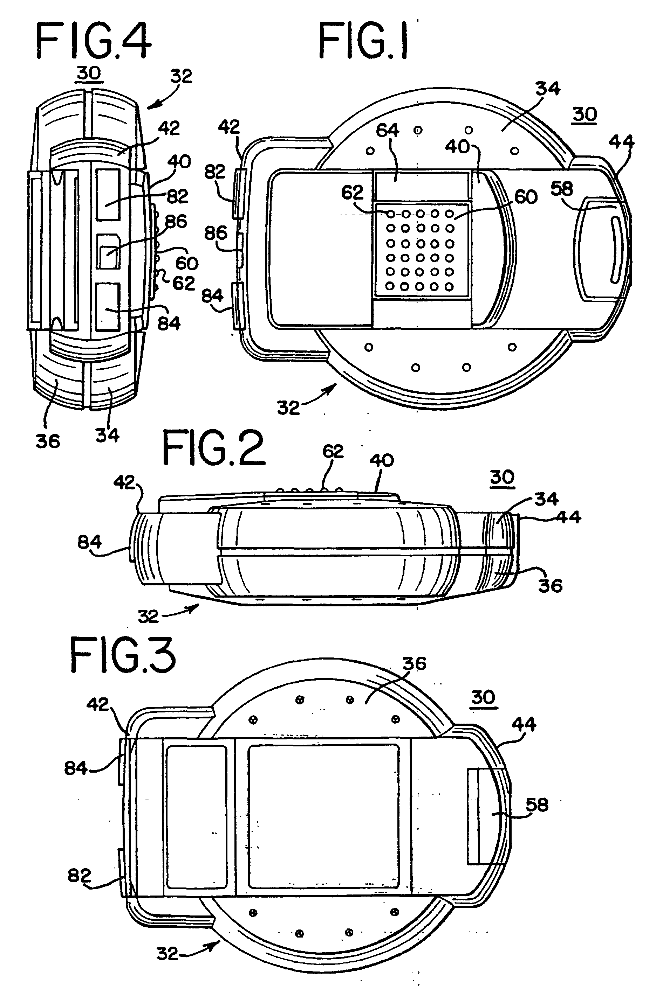

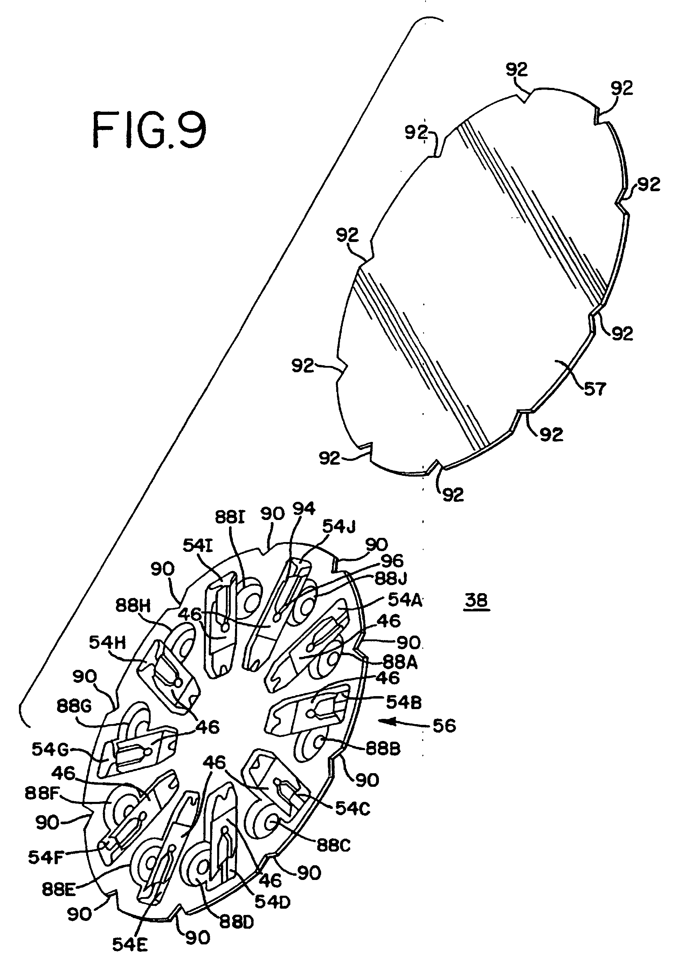

Referring now more specifically to FIGS. 1-4 of the drawings, therein is disclosed a blood glucose sensor dispensing instrument generally designated by the reference numeral 30 and embodying the present invention. The sensor dispensing instrument 30 includes an outer housing 32 having an upper case 34 and a lower case 36. The upper case 34 is pivotable with respect to the lower case 36 in a clam shell fashion so that a sensor pack 38 (FIG. 9) can be positioned within the housing 32. Once the sensor pack 38 is disposed within the housing 32, a slide actuator 40 on the upper case 34 of the housing 32 can be moved from a standby position (FIGS. 1-4) adjacent a rear end 42 of the upper case 34 toward an actuated or testing position adjacent a forward or testing end 44 of the upper case 34.

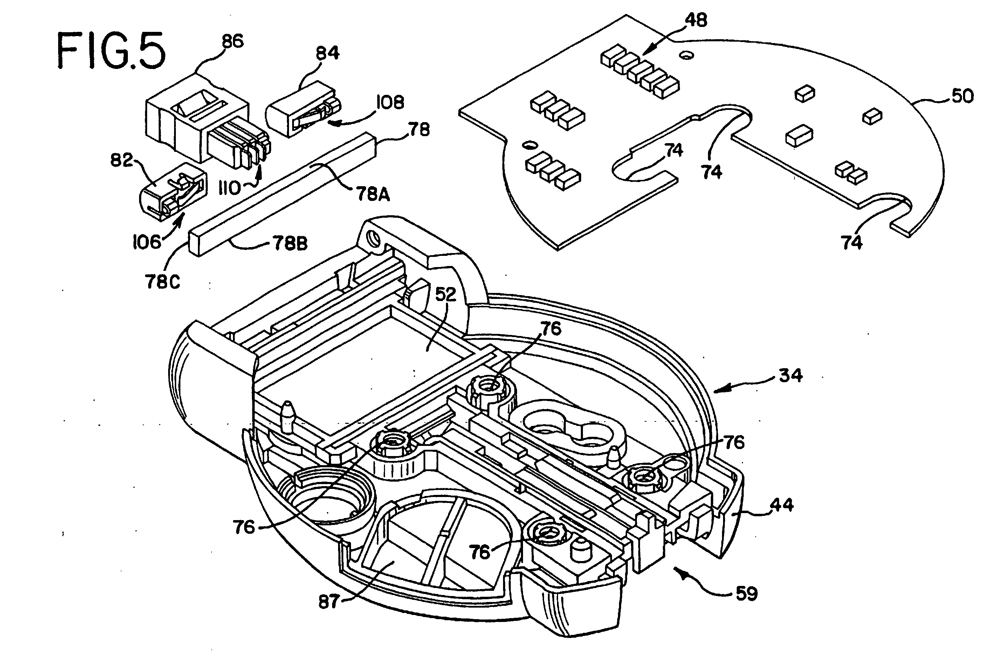

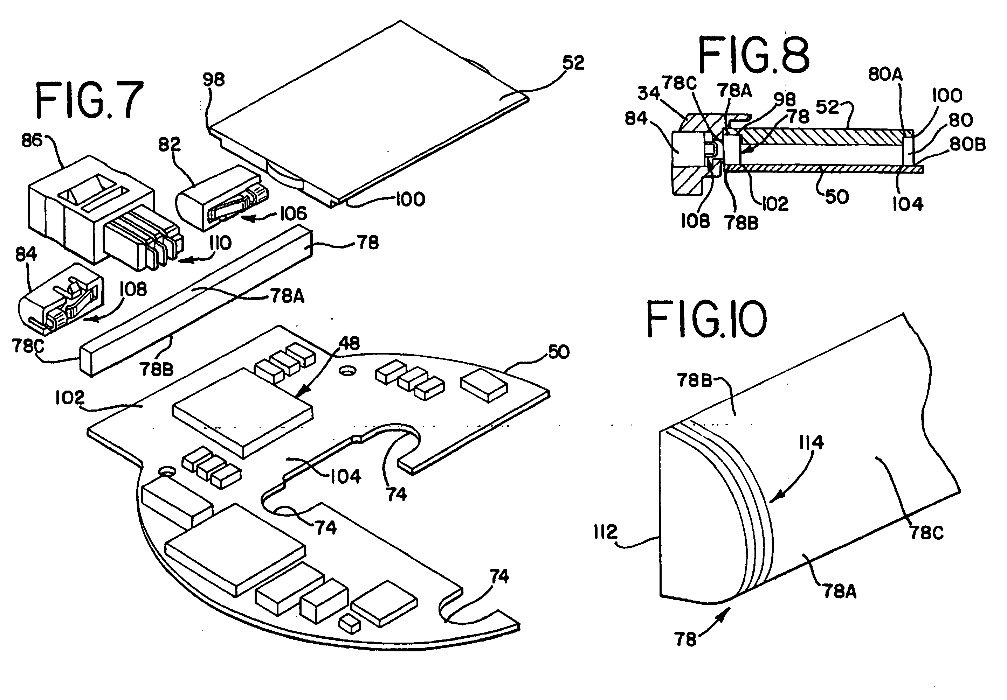

As the slide actuator 40 is moved toward its actuated position, the sensor pack 38 is rotated so that one of a plurality of sensors 46 (FIG. 9) disposed in the sensor pack 38 is ejected from the sensor pack 38 and is forced toward the testing end 44 of the upper case 34. Once the ejected sensor 46 has become lodged in its testing position projecting out from the testing end 44, contacts on the sensor 46 are coupled to electronic circuitry (generally designated as 48 in FIGS. 5 and 7) on a printed circuit board 50 disposed in the upper case 34. The circuitry 48 on the printed circuit board 50 may include a microprocessor or the like for processing, storing and/or displaying on a display screen 52 data generated during a blood glucose test procedure.

Once the blood analyzing test is completed, the slide actuator 40 is manually retracted in the opposite direction towards its standby position adjacent the rear end 42 of the upper case 34 and the sensor 46 can be removed from the housing 32. The sensor pack 38 is also rotated so that the next one of sensor cavities 54A-J disposed in a base portion 56 of the sensor pack 38 becomes positioned so that the sensor 46 in the next one of the sensor cavities 54A-J can be used in the next blood glucose testing procedure. As is discussed in more detail hereinafter, the sensor base portion 56 is covered by a foil 57 to seal the individual sensor cavities 54A-J.

As is illustrated in FIGS. 1-4, the upper case 34 and the lower case 36 of the sensor dispensing housing 32 are complementary, generally round in shape hollow containers that are adapted to be pivoted with respect to each other. The upper case 34 and the lower case 36 are maintained in their closed configuration as shown in FIGS. 1-4 by a latch 58 that extends from the lower case 36 and latches into a recess 59 -(FIGS. 5-6) at the forward end 44 of the upper case 34.

As previously indicated, the slide actuator 40 is mounted on the upper case 34 and can be slid between a standby position near the rear end 42 as shown in FIGS. 1, 2, and 4 and a testing position adjacent the forward end 44. When the slide actuator 40 is moved toward the forward end 44, the display screen 52 can be viewed from the top of the upper case 34. A slide latch 60 having a plurality of raised nubs 62 that provides a surface facilitating the movement of the slide latch 60 and the slide actuator 40 by a person using the sensor dispensing instrument 30 is used to control the movement of the slide actuator 40 and is used to place the instrument 30 in one of two operating modes. In a first or testing mode, the slide latch 60 is positioned as shown in FIGS. 1, 2 and 4. In a second or data processing mode, the slide latch 60 is slid laterally with respect to the slide actuator 40 into a recess area 64 in the slide actuator 40. In this regard, the movement of the slide actuator 40 towards the forward end 44 when the slide latch 60 is in its testing mode position results in the activation of the display screen 52 and permits the display screen 52 to be observed from the top of the upper case 34 of the instrument housing 32.

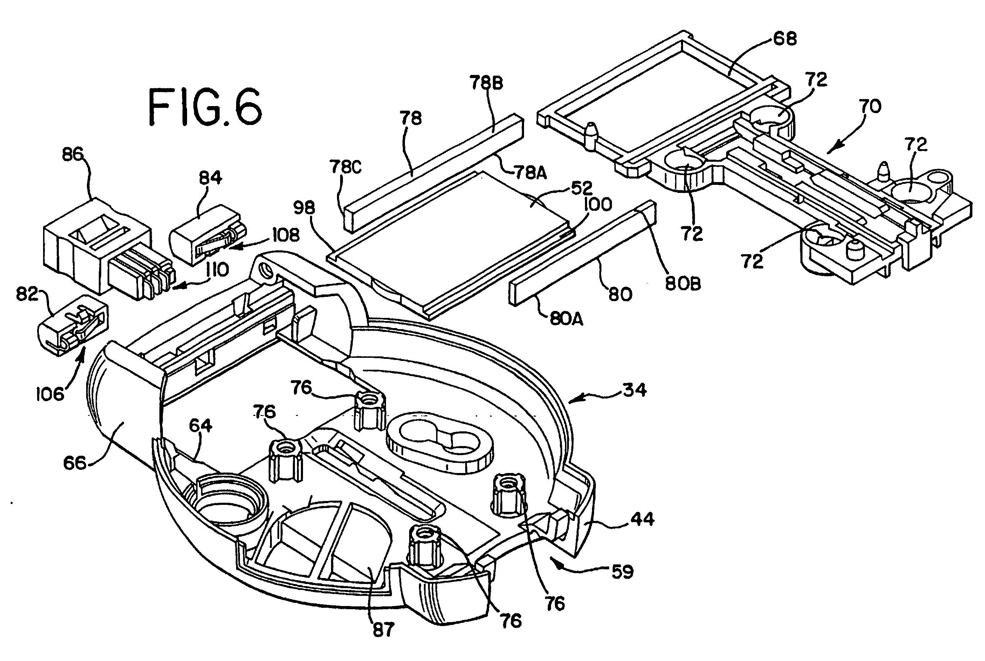

The upper case 34 includes a rectangular opening 64 (FIG. 6) in a rear section 66. The display screen 52 (which in the disclosed embodiment may be a liquid crystal display unit) is disposed in an rectangular shaped opening 68 in a housing guide 70. The housing guide 70 is retained within the upper case 34 by appropriate fasteners (not shown) that extend through securing holes 72 in the housing guide 70 and through notches 74 in the printed circuit board 50 so as to be secured in retaining holes 76 in the upper case 34. With the housing guide 70 so secured in the upper case 34, the display screen 52 is visible externally of the upper case 34 through the opening 64 when the slide actuator 40 is moved away from the rear end 42 of the upper case 34 toward the forward end 44 thereof. The liquid crystal display unit 52 needs to be coupled to the electronic circuits 48 on the printed circuit board 50 and as is discussed in more detail hereinafter, is so coupled via an elastomeric connector 78 and a flexible connector 80.

The displays appearing on the liquid crystal display unit 52 when the instrument 30 is in its data processing or display mode are controlled by manually actuated buttons 82 and 84 that project from the rear end 42 of the upper housing case 34 so as to be accessible from the external portion of the upper case 34. For example, the buttons 82 and 84 can be depressed to view and/or input the testing information that will be displayed on the liquid crystal display unit 52. Additionally, a data port connector 86 can be provided in the rear end 42 of the upper case 34 between the buttons 82 and 84. The data port connector 86 also is accessible from the external portion of the upper case 34. As is also discussed in further detail hereinafter, the buttons 82 and 84 and the data port connector 86 are coupled to the electronic circuits 48 on the printed circuit board 50 via the elastomeric connector 78.

The upper case 34 also contains batteries (not shown) that are retained in a battery holder (not shown) that is positioned in a battery holder slot 87 (FIGS. 5-6). The batteries provide power for the electronic components within the instrument 30 including the circuitry 48 on the printed circuit board 50 and the liquid crystal display unit 52.

The sensor pack 38 is formed of the circularly shaped base portion 56 and the correspondingly configured foil 57. The sensor cavities 54A-J are formed as depressions in the base portion 56 with each of the sensor cavities 54A-J adapted to house one of the sensors 46. Each of the sensor cavities 54A-J is in fluid communication with a corresponding one of desiccant cavities 88A-J. Each of the desiccant cavities 88A-J is formed of a small depression in the base portion 56 adjacent the corresponding one of the sensor cavities 54A-J. Desiccant material is disposed in the desiccant cavities 88A-J in order to insure that the sensor cavities 54A-J are maintained at an appropriate humidity level so that the reagent material in the sensor 46 disposed in the particular sensor slot 54A-J is not adversely affected prior to being used. The desiccant material might be in the form of a small bag or round bead of material or any other form that can be readily disposed in the desiccant cavities 88A-J. The amount of such desiccant material placed in each of the desiccant cavities 88A-J will be dependent on the amount that is required to maintain the sensor cavities 54A-J in a desiccate state.

The notches 90 are formed along the outer peripheral edge of the base portion 56. When the foil 57 is sealed to the base portion 56, the notches 92 along the outer peripheral edge of the foil 57 will be in alignment with the notches 90 to thereby form an integral series of notches along the outer peripheral edge of the sensor pack 38. Each of the notches formed by the notches 90 and 92 is associated with one of the sensor cavities 54A-J in the base portion 56 such that these notches can be used to advance (rotate) the sensor pack 38 as the slide actuator 40 is being returned to its standby position near the rear end 42 of the upper case 34.

The foil 57 is adapted to cover the top of the base portion 56 and be affixed to the base portion 56 by heat sealing along the entire outer peripheral edge of the foil 57 to the outer peripheral edge of the base portion 56. The foil 57 also is heat sealed about the entire perimeter of each set of the sensor retaining cavities 54A-J and the desiccant cavities 88A-J to seal the sensor retaining cavities 54A-J and the desiccant cavities 88A-J such that the individual sensors 46 are maintained in a desiccated state and isolated from each other. As a result, the opening of one of the sensor cavities 54A-J will not affect the desiccated state of any of the other sensor cavities 54A-J.

As is illustrated with respect to the sensor 46 disposed in the cavity 54J of the sensor pack 38 (FIG. 9), each of the sensors 46 is provided with a capillary channel that extends from a front, testing end 94 of the sensor 46 to biosensing or reagent material disposed in the sensor 46. When the testing end 94 of the sensor 46 is placed into fluid (for example, blood that is accumulated on a person's finger after the finger has been pricked), a portion of the fluid is drawn into the capillary channel by capillary action so that a sufficient amount of fluid to be tested is drawn into the sensor 46. The fluid then chemically reacts with the reagent material in the sensor 46 so that an electrical signal indicative of the blood glucose level in the blood being tested is supplied to the contacts 96 that are coupled to the electrically circuits 48 on the printed circuit board 50.

In order for the sensor dispensing instrument 30 to be used by a person for testing of blood glucose, the instrument 30 needs to be opened so that the sensor pack 38 can be inserted into the interior of the upper case 34 of the instrument housing 32. The instrument 30 is opened by moving the latch 58 away from its latching position in the recess 59 of the upper case 34. With the latch 58 so released, the lower case 36 can be swung away from the upper case 34, a previously used sensor pack 38 can be removed, and a new sensor pack 38 can be positioned in the upper case 34. With the sensor pack 38 so installed, the lower case 36 can be swung toward the upper case 34 and latched to the upper case 34 by pivoting the latch 58 into the recess 59.

With the upper case 34 and the lower case 36 latched together, the slide latch 60 can be manipulated to place the sensor dispensing instrument 30 in either its display or data processing mode or its testing mode. For example, the slide latch 60 can be moved laterally with respect to the longitudinal axis of the instrument 30 into the recess 64 to place the instrument 30 in its display mode. When the slide latch 60 is moved laterally in this manner, the movement of the slide actuator 40 toward the front end 44 results in the sensor dispensing instrument 30 being activated into its display mode. In addition, the movement of the slide actuator 40 toward the front end 44 allows the liquid crystal display unit 52 to be visible from above the upper case 34. Information concerning the sensor pack 38 and the tests that have been conducted can be displayed on the liquid crystal display unit 52. The information that is displayed can be controlled by the actuation of the buttons 82 and 84 projecting from the rear end 42 of the upper case 34. The buttons 82 and 84 also are used to input information into the circuitry 48 on the printed circuit board 50 in accordance with the displays on the liquid crystal display unit 52.

When the user of the sensor dispensing instrument 30 has completed obtaining information or inputting data, the slide actuator 40 is returned toward the rear end 42 of the upper case 34 by pushing on the nubs 62 projecting from the slide latch 60. The instrument 30 will then be in its off or standby condition. With the slide actuator 40 in its fully retracted, standby position, the slide actuator 40 will again cover the liquid crystal display unit 52 and the slide latch 60 can be moved laterally back to its normal position as illustrated in FIG. 1 of the drawings.

The primary use of the sensor dispensing instrument 30 is in connection with a blood glucose test. When such a test is to be conducted, a user of the instrument 30 can push against the nubs 62 on the slide latch 60 in order to slide the slide actuator 40 from its standby position near the rear end 42 toward the front or testing end 44. As the slide actuator 40 is moved toward the testing end 44, the sensor dispensing instrument 30 is placed into its testing mode.

The forward movement of the slide actuator 40 results in one of the sensors 46 being ejected from the one of the sensor cavities 88A-J in the sensor pack 38 installed in the upper case 34. The ejected sensor 46 is guided within the upper case 34 until the front or testing end 94 of the sensor 46 is projecting out from the front end 44 of the upper case 34 through the latch 58. Once the sensor 46 is in its testing position, the testing end 94 of the sensor 46 then can be placed into fluid, such as blood that is accumulated on a person's finger after the finger has been pricked. The fluid is absorbed into the sensor 46 and chemically reacts with the reagent material in the sensor 46 so that an electrical signal indicative of the blood glucose level in the blood being tested is supplied to the contacts 96 and to the electrical circuits 48 on the printed circuit board 50.

Once the blood analyzing test is completed, the slide actuator 40 is moved away from the front end 44 toward the rear end 42 of the upper case 34 by a user of the instrument 30 pushing on the nubs 62 projecting from the slide latch 60. As the slide actuator 40 is moved toward the rear end 42, the sensor pack 46 is rotated by means of the notches 90, 92 in order to place another one of the sensor cavities 54A-J in proper alignment so that another one of the sensors 46 in the sensor pack 38 can be ejected from the sensor pack 38 and used in another testing procedure.

As can be appreciated, the sensor dispensing instrument 30 needs to be relatively compact in size so that it can easily be carried and handled by a person using the dispensing instrument 30. Nevertheless, the sensor dispensing instrument 30 needs to include the mechanical elements needed to advance (rotate) the sensor pack 38 disposed therein, to eject individual ones of the sensors 46 from the individualized sensor cavities 54A-J in the sensor pack 38 and to move the ejected sensor 46 into a testing position. In addition, the instrument 30 has to include a microprocessor or other data processing circuitry 48 on the printed circuit board 50 to obtain the data generated by the sensor 46 during a test procedure, to process inputs from the manually actuated buttons 82 and 84 to provide information with respect to the test being conducted and to provide the accumulated information to the display screen 52 and/or to provide the accumulated information to other analyzing or computer equipment via the data port connector 86.

The data processing circuitry 48 on the printed circuit board 50 needs to be coupled to the display screen 52 as well as to the manually actuated input buttons 82 and 84 and the data port connector 86. In order to so couple those components in the limited amount of space available in the relatively compact sized fluid sensor dispensing instrument 30, the elastomeric connector 78 is used to provide the electrical connections to the data processing circuitry 48 from the display screen 52, the manually actuated buttons 82 and 84 and the data port connector 86.

As is shown in particular in FIGS. 7-8, the printed circuit board 50 and the display screen 52 lie in generally parallel planes with the connectors 78 and 80 sandwiched between the printed circuit board 50 and the display screen 52 so as to keep the display screen 52 spaced slightly apart from the printed circuit board 50. The display screen 52 is a generally flat liquid crystal diode display that has circuit traces or contacts along opposite ledges 98 and 100. As is illustrated in FIG. 8, a side 78A of the connector 78 is in contact with the circuit traces on the ledge 98 and a side 80A of the connector 80 is in contact with the circuit traces on the ledge 100. An opposite side 78B of the connector 78 is in contact with circuit traces on the printed circuit board 50 along an edge area 102 and an opposite side 80B of the connector 80 is in contact with additional circuit traces on the printed circuit board 50 along an area 104. The connectors 78 and 80 thereby are sandwiched between the display screen 52 and the printed circuit board 50 such that the display screen 52 and the printed circuit board 50 apply a compression force against the elastomeric connectors 78 and 80 and the connectors 78 and 80 make sufficient contact with the display screen 52 and the printed circuit board 50 to couple electrically the display screen 52 to the electrical circuits 48 on the printed circuit board 50.

The connector 78 additionally electrically couples the manually actuated buttons 82 and 84 and the data port connector 86 to the circuit traces along the edge area 102 of the printed circuit board 50. The button 82 includes contacts 106, the button 84 includes contacts 108 and the data port connector 86 includes contacts 110. The contacts 106 or 108 are normally spaced apart from the side 78C of the connector 78 and will only make contact with the side 78C of the connector 78 when their respective buttons 82 and 84 are depressed by a user of the instrument 30 in order to input into or obtain information from the electrical components 48 on the printed circuit board 50. On the other hand, the contacts 110 extending from the data port connector 86 normally are in contact with the side 78C of the connector 78 so that the data port connector 86, which may be a modular telephone jack type connector, is normally coupled to the electrical components 48 on the printed circuit board 50.

Due to the relatively limited space available for the interconnection of the buttons 82 and 84, the data port connector 86 and the display screen 52 to the printed circuit board 50, the connector 78 needs to be mounted between those components without occupying an excessive amount of space and in addition needs to be able to couple the display screen 52 and the printed circuit board 50 that lie in parallel planes and to couple the contacts 106 and 108 extending respectively from the buttons 82 and 84 and the contacts 110 extending from the data port connector 86 in a direction that is generally parallel to the planes of the display screen 52 and the printed circuit board 50.

In order to accomplish these connections in the limited space available, the connector 78 is an elastomeric connector having a generally silicone sponge rubber core 112 with the contacting sides 78A and 78B being in generally parallel planes and the contacting side 78C being a generally a right angle to the planes of the sides 78A and 78B. As is illustrated in FIG. 10, the edges of the side 78C having a slight radius and generally U-shaped metal conductive traces or filaments 114 (some of which are illustrated in FIG. 10) extend' about the core 112 at least on the sides 78A, 78B and 78C. As a result, the connector 78 enables the coupling of the components (i.e., the display screen 52, the printed circuit board 50 and the contacts 106, 108 and 110) that lie on three different sides of the connector 78. Moreover, the fine pitch between the metal filaments provides more conducting paths for coupling the display screen 52, the contacts 106 and 108 extending from the buttons 82 and 84 and the contacts 110 extending from the data port connector 86 to the electrical traces on the edge area 102 of the printed circuit board 50. The fact that the connector 78 is formed from a sponge rubber substance permits the connector 78 to be sandwiched between and thereby retained in position between the display screen 52 and the printed circuit board 50 due to the force exerted on the side 78A of the connector 78 by the display screen 52 and the side 78B of the connector 78 by the printed circuit board 50. As a result, the connector 78 also is maintained in position with respect to the contacts 106, 108 and 110 so that a reliable contact is maintained between the display screen 52, the printed circuit board 50 and the contacts 106, 108 and 110 without the necessity of spacing consuming holders or retainers.

One type of elastomeric connector that can be used as the connector 78 is a Shin-Etsu Shin-Flex GBU connector. Such a connector has a core 112 of soft silicone rubber with the U-shaped metal filaments 114 made of gold-plated brass and wrapped around the core 112. One of the advantages of that particular connector is that the filaments 114 are only .04 millimeters in diameter and have a pitch or separation of only .10 millimeters so that the contacts 106, 108 and 110 and the circuits on the display screen 52 can be readily coupled to the circuits 48 on the printed circuit board 50.

On the other hand, the connector 80 only needs to couple the traces at the ledge 100 of the display screen 52 to the printed circuit board 50. A connector that can be used for coupling such a display screen 52 to a printed circuit board 50 is a Shin-Etsu Shin-Flex SS connector. This connector consists of alternating segments of conducting and non-conducing solid silicone rubber sandwiched between two walls of soft silicone rubber. The advantages of this type of connector is that it does not require space consuming retainers or the like to maintain it between the display screen 52 and the printed circuit board 50 and only requires slight pressure to make a reliable contact because of the soft silicone rubber insulating walls.

While the invention has been described with reference to details of the illustrated embodiment, these details are not intended to limit the scope of the invention as defined in the appended claims. For example, the instrument 30 can be used for testing fluids other than blood glucose. In fact, the instrument 30 can be used in connection with analyzing any type chemistry fluid that can be analyzed by means of a reagent material.

## Claims

1. A sensor dispensing instrument (30) for handling a plurality of fluid sensors (46) retained in a sensor pack (38), said sensors (46) generating electrical data during a fluid test, said instrument (30) comprising:
an outer housing (32);
a display means (52) in said housing (32) for displaying information;
a circuit means (50) disposed in said housing (32) adjacent said display means (52) and having data processing means thereon to receive said electrical data generated by said sensors (46);
data means (82, 84, 86) accessible from externally of said housing (32) and being electrically coupled to said circuit means (50); and
a flexible connector means (78) disposed between said display means (52) and said circuit means (50) and adjacent to said data means (82, 84, 86) for coupling said display means (52) and said data means (82, 84, 86) to said circuit means (50), said connector means (78) having conductors on at least first (78A) and second (78B) opposed sides and a third side (78C) extending therebetween with said display means (52) electrically coupled to said first side (78A) of said connector means (78), said circuit means (50) electrically coupled to said second side (78B) of said connector means (78), and said data means (82, 84, 86) being electrically coupled to said third side (78C) of said connector means (78),
wherein said connector means (78) includes a soft core (112) and wherein said conductors on said connector means includes a plurality of conductive filaments (114),
said conductive filaments (114) being metallic, generally U-shaped conductors extending about said first (78A), second (78B) and third (78C) sides of said core (112).

2. A sensor dispensing instrument (30) as set forth in claim 1 wherein said soft core ( 112) is a soft silicone rubber.

3. A sensor dispensing instrument (30) as set forth in claim 1 or 2 wherein said display means (52) and said circuit means (50) lie in generally parallel planes and said connector means (78) is sandwiched between said display means (52) and said circuit means (50) such that said connector means (78) is retained between and electrically couples said display means (52) and said circuit means (50).

4. A sensor dispensing instrument (30) as set forth in claim 3 wherein said data means (82, 84, 86) includes contacts (106, 108, 110) extending therefrom in a direction that is generally parallel to the planes of said display means (50) and said circuit means (52) and wherein said third side (78C) of said connector means (78) is generally at a right angle with respect to said first (78A) and second (78B) sides of said connector means (78).

5. A sensor dispensing instrument (30) as set forth in claim 3 or 4 including an additional flexible connector (80) sandwiched between said display means (52) and said circuit means (50) and providing electrical coupling between said display means (52) and said circuit means (50), said additional connector (80) and said connector means (78) being disposed at opposite edges (98,100) of said display means (52).

6. A sensor dispensing instrument (30) as set forth in one of the claims 1 to 5 wherein said display means (52) is a liquid crystal display.

7. A sensor dispensing instrument (30) as set forth in one of the claims 1 to 6 wherein said circuit means (50) is a printed circuit board (48) having data processing means thereon.

8. A sensor dispensing instrument (30) as set forth in one of the claims 1 to 7 wherein said data means (82, 84, 86) is a plurality of manually actuated buttons for inputting information into said circuit means (50) or obtaining information from said circuit means (50).

9. A sensor dispensing instrument (30) as set forth in one of the claims 1 to 8 wherein said data means (82, 84, 86) includes an electrical connector (106, 108, 110) coupled to said circuit means (50) through said connector means (78).

## Patentansprüche

1. Sensor-Ausbringgerät (30) zur Handhabung einer Vielzahl von Flüssigkeitssensoren (46), die in einer Sensorpackung (38) gehalten werden, wobei die genannten Sensoren (46) elektrische Daten während eines Flüssigkeitstest erzeugen und das genannte Gerät (30) umfasst:
ein Aussengehäuse (32);
ein Anzeigemittel (52) im genannten Gehäuse (32) zur Anzeige und Wiedergabe von Information;
ein Schaltkreismittel (50), das im genannten Gehäuse (32) in Nachbarschaft zum genannten Anzeigemittel (52) angeordnet ist und darauf Datenverarbeitungsmittel aufweist, um die durch die genannten Sensoren (46) erzeugten elektrischen Daten aufzunehmen;
Datenmittel (82, 84, 86), die von ausserhalb des genannten Gehäuses (32) zugänglich sind und elektrisch an das genannte Schaltkreismittel (50) angeschlossen werden; und
ein biegsames Steckermittel (78), das zwischen dem genannten Anzeigemittel (52) und dem genannten Schaltkreismittel (50) und in Nachbarschaft zu den genannten Datenmitteln (82, 84, 86) zum Anschluss des genannten Anzeigemittels (52) und der genannten Datenmittel (82, 84, 86) an das genannte Schaltkreismittel (50) angeordnet ist, wobei das genannte Steckermittel (78) Leiter auf mindestens ersten (78A) und zweiten (78B) gegenüberliegenden Seiten und auf einer dritten Seite (78C) aufweist, die sich dazwischen erstreckt, wobei das genannte Anzeigemittel (52) elektrisch an die genannte erste Seite (78A) des genannten Steckermittels (78), das genannte Schaltkreismittel (50) elektrisch an die genannte zweite Seite (78B) des genannten Steckermittels (78) und die genannten Datenmittel (82, 84, 86) elek-trisch an die genannte dritte Seite (78C) des genannten Steckermittels (78) angeschlossen werden,
worin das genannte Steckermittel (78) einen Weichkern (112) und die genannten Leiter auf dem genannten Steckermittel eine Vielzahl leitfähiger Filamente (114) einschließen,
wobei die genannten leitfähigen Filamente (114) metallische, im Allgemeinen U-förmige Leiter sind, die sich um die genannten ersten (78A), zweiten (78B) und dritten (78C) Seiten des genannten Kerns (112) erstrecken.

2. Sensor-Ausbringgerät (30) gemäß Anspruch 1, worin der genannte Weichkern (112) ein Weichsilicongummi ist.

3. Sensor-Ausbringgerät (30) gemäß Anspruch 1 oder 2, worin das genannte Anzeigemittel (52) und das genannte Schaltkreismittel (50) in im Allgemeinen parallelen Ebenen liegen und das genannte Steckermittel (78) als Sandwich zwischen dem genannten Anzeigemittel (52) und dem genannten Steckermittel (50) so angeordnet ist, dass das genannte Steckermittel (78) zwischen dem genannten Anzeigemittel (52) und dem genannten Schaltkreismittel (50) gehalten und elektrisch an diese angeschlossen wird.

4. Sensor-Ausbringgerät (30) gemäß Anspruch 3, worin die genannten Datenmittel (82, 84, 86) Kontakte (106, 108, 110) einschließen, die sich daraus in einer Richtung erstrecken, die im Allgemeinen parallel zu den Ebenen des genannten Anzeigemittels (52) und des genannten Schaltkreismittels (50) verläuft, und worin die genannte dritte Seite (78C) des genannten Steckermittels (78) im Allgemeinen in einem rechten Winkel bezüglich der genannten ersten (78A) und zweiten (78B) Seiten des genannten Steckermittels (78) verläuft.

5. Sensor-Ausbringgerät (30) gemäß Anspruch 3 oder 4, das einen zusätzlichen biegsamen Stecker (80) einschließt, der zwischen dem genannten Anzeigemittel (52) und dem genannten Schaltkreismittel (50) als Sandwich angeordnet ist und den elektrischen Anschluss zwischen dem genannte Anzeigemittel (52) und dem genannten Schaltkreismittel (50) herstellt, wobei der genannte zusätzliche Stecker (80) und das genannte Steckermittel (78) an gegenüberliegenden Kanten (98, 100) des genannten Anzeigemittels (52) angeordnet werden.

6. Sensor-Ausbringgerät (30) gemäß einem der Ansprüche 1 bis 5, worin das genannte Anzeigemittel (52) eine Flüssigkristall-Anzeige ist.

7. Sensor-Ausbringgerät (30) gemäß einem der Ansprüche 1 bis 6, worin das genannte Schaltkreismittel (50) ein gedrucktes Schaltkreisbrett (48) ist, das darauf Datenverarbeitungsmittel aufweist.

8. Sensor-Ausbringgerät (30) gemäß einem der Ansprüche 1 bis 7, worin die genannten Datenmittel (82, 84, 86) durch eine Vielzahl von von Hand betätigten Knöpfen zur Eingabe von Information in das genannte Schaltkreismittel (50) oder zum Erhalt von Information aus dem genannten Schaltkreismittel (50) dargestellt werden.

9. Sensor-Ausbringgerät (30) gemäß einem der Ansprüche 1 bis 8, worin die genannten Datenmittel (82, 84, 86) einen Elektrostecker (106, 108, 110) einschließen, die an das genannte Schaltkreismittel (50) durch das genannte Steckermittel (78) gekoppelt und angeschlossen werden.

## Revendications

1. Dispositif de distribution pour détecteurs (30) pour la manipulation d'une pluralité dé détecteurs pour fluide (46) contenus dans un paquet de détecteurs (38), lesdits détecteurs (46) générant dès données électriques durant un test de fluide, ledit instrument (30) comprenant:
un boîtier extérieur (32);
un moyen d'affichage (52) dans ledit boîtier (32) pour afficher des informations;
un moyen de circuit (50) disposé dans ledit boîtier (32) adjacent audit moyen d'affichage (52) et ayant des moyens de traitement de données afin de recevoir lesdites données électriques générées par lesdits détecteurs (46);
des moyens de données (82, 84, 86) accessibles de l'extérieur dudit boîtier (32) et connectés électriquement audit moyen de circuit (50); et
un moyen de connexion flexible (78) disposé entre ledit moyen d'affichage (52) et ledit moyen de circuit (50) et adjacent auxdits moyens de données (82, 84, 86) pour coupler ledit moyen d'affichage (52) et lesdits moyens de données (82, 84, 86) audit moyen de circuit (50), ledit moyen de connexion (78) ayant des conducteurs au moins sur les premier (78A) et deuxième (78B) côtés opposés et un troisième côté (78C) s'étendant entre eux, ledit moyen d'affichage (52) étant connecté électriquement audit premier côté (78A) dudit moyen de connexion (78), ledit moyen de circuit (50) étant connecté électriquement audit deuxième côté (78B) dudit moyen de connexion (78) et lesdits moyens de données (82, 84, 86) étant connectés électriquement audit troisième côté (78C) dudit moyen de connexion (78),
dans lequel ledit moyen de connexion (78) inclut un noyau doux (112) et dans lequel lesdits conducteurs sur ledit moyen de connexion incluent une pluralité de filaments conducteurs (114),
lesdits filaments conducteurs (114) étant métalliques, généralement des conducteurs en forme de U s'étendant autour desdits premier (78A), deuxième (78B) et troisième (78C) côtés dudit noyau (112).

2. Dispositif de distribution pour détecteurs (30) tel que décrit dans la revendication 1, dans lequel ledit noyau doux (112) est un caoutchouc mou à la silicone.

3. Dispositif de distribution pour détecteurs (30) tel que décrit dans la revendication 1 ou 2, dans lequel ledit moyen d'affichage (52) et ledit moyen de circuit (50) sont situés dans des plans essentiellement parallèles et ledit moyen de connexion (78) est pris en sandwich entre ledit moyen d'affichage (52) et ledit moyen de circuit (50), de telle façon que ledit moyen de connexion (78) soit retenu entre ledit moyen d'affichage (52) et ledit moyen de circuit (50) et les connecte électriquement.

4. Dispositif de distribution pour détecteurs (30) tel que décrit dans la revendication 3, dans lequel lesdits moyens de données (82, 84, 86) incluent des contacts (106, 108, 110) s'étendant depuis ceux-ci dans une direction qui est essentiellement parallèle aux plans dudit moyen d'affichage (50) et dudit moyen de circuit (52) et dans lequel ledit troisième côté (78C) dudit moyen de connexion (78) est essentiellement à angle droit par rapport auxdits premier (78A) et deuxième (78B) côtés dudit moyen de connexion (78).

5. Dispositif de distribution pour détecteurs (30) tel que décrit dans la revendication 3 ou 4, incluant un connecteur flexible supplémentaire (80) pris en sandwich entre ledit moyen d'affichage (52) et ledit moyen de circuit (50) et fournissant une connexion électrique entre ledit moyen d'affichage (52) et ledit moyen de circuit (50), ledit connecteur supplémentaire (80) et ledit moyen de connexion (78) étant disposés sur des bords opposés (98, 100) dudit moyen d'affichage (52).

6. Dispositif de distribution pour détecteurs (30) tel que décrit dans une des revendications 1 à 5, dans lequel ledit moyen d'affichage (52) est un écran à cristaux liquides.

7. Dispositif de distribution pour détecteurs (30) tel que décrit dans une des revendications 1 à 6, dans lequel ledit moyen de circuit (50) est une carte de circuits imprimés (48) portant des moyens de traitement de données.

8. Dispositif de distribution pour détecteurs (30) tel que décrit dans une des revendications 1 à 7, dans lequel lesdits moyens de données (82, 84, 86) sont une pluralité de boutons actionnés manuellement pour introduire des informations dans ledit moyen de circuit (50) ou obtenir des informations dudit moyen de circuit (50).

9. Dispositif de distribution pour détecteurs (30) tel que décrit dans une des revendications 1 à 8, dans lequel lesdits moyens de données (82, 84, 86) comprennent un connecteur électrique (106, 108, 110) couplé audit moyen de circuit (50) par l'intermédiaire dudit moyen de connexion (78).
